# EUROPEAN PATENT SPECIFICATION

(11) **EP 2 173 393 B1**
(45) Date of publication and mention of the grant of the patent: **12.03.2014**
(21) Application number: 08781567.6
(22) Date of filing: 09.07.2008
(51) Int. Cl.: A61L 31/02, A61L 31/08, A61L 31/14, A61L 31/12

(54) **BORON-ENHANCED SHAPE MEMORY ENDOPROSTHESES**
BORON-VERSTÄRKTE FORMSPEICHERNDE ENDOPROTHESEN
ENDOPROTHÈSES À MÉMOIRE DE FORME RENFORCÉES DE BORE

(30) Priority: 13.07.2007 US 777729
(43) Date of publication of application: 14.04.2010
(73) Proprietor: Boston Scientific Scimed, Inc., Maple Grove, MN 55311-1566 (US)
(72) Inventor: WEBER, Jan, NL-6228 GJ Maastricht (NL); O'BRIEN, Barry, Galway (IE)
(74) Representative: Peterreins, Frank
(86) International application number: PCT/US2008/069536
(87) International publication number: WO 2009/012099

(56) References cited:
- EP-A2- 1 685 861
- WO-A1-2007/024537
- WO-A2-2004/108021
- US-A- 5 477 864
- US-A1- 2004 187 980
- US-A1- 2007 137 734
- RIBEIRO R ET AL: "Tribological characteristics of boronized niobium for biojoint applications" VACUUM, PERGAMON PRESS, GB, vol. 80, no. 11-12, 7 September 2006 (2006-09-07), pages 1341-1345, XP025009628 ISSN: 0042-207X [retrieved on 2006-09-07]
- YANG W S ET AL: "Ductilization of Ti-Ni-Pd shape memory alloys with boron additions" SCRIPTA METALLURGICA ET MATERIALIA, OXFORD, GB, vol. 28, no. 2, 15 January 1993 (1993-01-15), pages 161-165, XP024182201 ISSN: 0956-716X [retrieved on 1993-01-15]

## Description

### TECHNICAL FIELD

This invention relates to stents.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism can include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

US200/4187980 A1 relates to a shape-memory alloy comprising in combination a shape memory alloy and a coherent nanodispersion of an additional phase providing a misfit of less than about 2.5% in the lattice structure between the nanodispersion and the parent phase. These alloys are contemplated to be used in stents. Minor amounts of boron can be optionally added to a TiNi based alloy.

### SUMMARY

In one aspect, an endoprosthesis is disclosed that can include a member capable of supporting a body passageway. The member can have a surface region overlying a bulk region, and can include a shape memory alloy and boron. The concentration of boron in the surface region can be greater than the concentration of boron in the bulk region.

In some embodiments, the shape memory alloy can include titanium, niobium, or a combination thereof. In some embodiments, the shape memory alloy can include a Ni-Ti alloy.

In some embodiments, the surface region can include boride intermetallic phases. For example, the surface region can include titanium boride phases, niobium boride phases, or a combination thereof. In some embodiments, the surface region can be at least I nanometer thick and/or no more than 5 microns thick. In some embodiments, the surface region can have a concentration at least 1 weight percent boron and a thickness of between 1 nanometer and 3 microns. The surface region can also have a surface region concentration of up to 30 weight percent boron and a thickness of between 1 nanometer and 3 microns. The concentration of boron in the member can decrease in the thickness direction from the surface region to the bulk region.

In some embodiments, the endoprosthesis can also include a carbon layer overlying layer the surface region, carbides embedded within the surface region, or a combination thereof. For example, titanium carbide can be embedded within the surface region.

In some embodiments, the member capable of supporting a body passageway can be a stent. For example, the stent can be a self-expanding stent and/or a superficial femoral artery stent. In some embodiments, the member can further include portions comprising the shape memory alloy but lacking boron.

In another aspect, a method of producing an endoprosthesis is disclosed that includes heat setting a shape member alloy endoprosthesis into a predetermined expanded state and implanting boron ions into the surface of the endoprosthesis. In some embodiments, the boron ions can be implanted by plasma ion immersion implantation.

In some embodiments, the boron ions can be selectively implanted into the surface of the heat set endoprosthesis to produce portions that include a surface comprising the shape memory alloy and boron, and portions that include a surface comprising the shape memory alloy but lacking boron.

In some embodiments, the method can further include incorporating carbon into or onto the surface of the heat set endoprosthesis.

Other features, objects, and advantages will be apparent from the description and drawings, and from the claims.

### DESCRIPTION OF DRAWINGS

FIG. 1 illustrates an exemplary stent having a boron enriched surface.
FIG. 2 illustrates a second exemplary stent having a boron enriched surface.
FIG. 3 illustrates a third exemplary stent having boron enriched portions and non-boron enriched portions.
FIG. 4 illustrates a fourth exemplary stent having boron enriched portions and non-boron enriched portions.
FIGS. 5A and 5B illustrate exemplary environments for implanting boron ions into the surface of a stent.

Like reference symbols in the various drawings indicate like elements.

### DETAILED DESCRIPTION

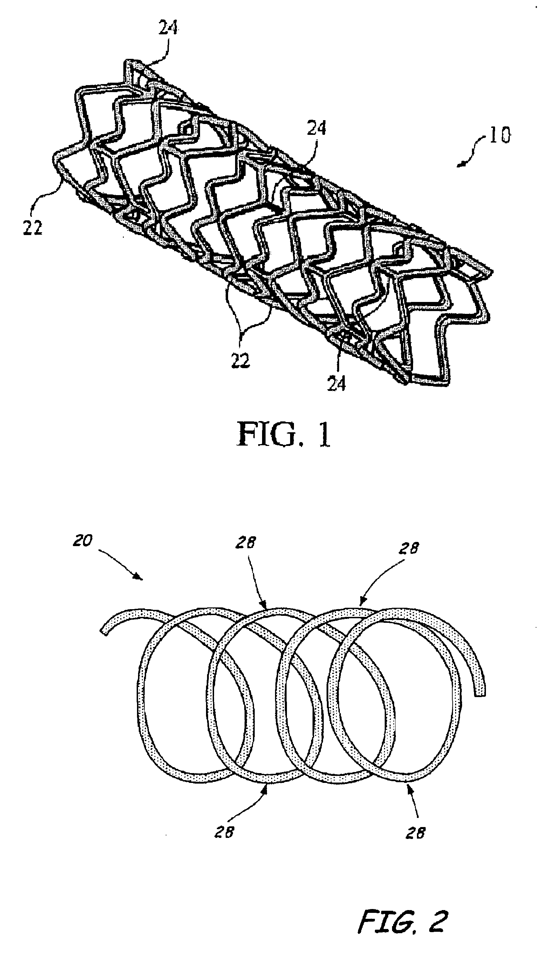

Referring to FIG. 1, a stent 10 can have the form of a tubular member defined by a plurality of bands 22 and a plurality of connectors 24 that extend between and connect adjacent bands. During use, bands 22 can expand from an initial, small diameter compressed state to a larger diameter to contact stent 10 against a wall of a vessel, thereby maintaining the patency of the vessel. Connectors 24 can provide stent 10 with flexibility and conformability that allow the stent to adapt to the contours of the vessel.

Stent 10 can include a shape memory alloy. The shape memory alloy can include titanium, niobium, or a combination thereof. For example, the shape memory alloy can be a Ni-Ti alloy, such as nitinol. Other examples of suitable shape memory alloys can include Ti-Pd alloys, Ti-Pd-Ni alloys, Ni-Ti-Cu alloys, Ti-Nb-Al alloys , Hf-Ti-Ni alloys, Ti-Nb, and Ni-Zr-Ti alloys.

Stent 10 can include a surface region and an underlying bulk region. Both regions can include the shape memory alloy. In addition, the concentration of boron can be greater in the surface region than in the bulk region, thereby creating a boron enriched surface. Boron or boron ions can be implanted using a number of techniques, some of which are discussed below. The Boron can be implanted in the surface to achieve a surface concentration of boron of up to 30 % atm. The concentration of boron in the overall stent mass can depend on the thickness of the stent. The implantation dose of Boron can be about 4.8 x 10(17) boron/cm² to achieve a 30 % atm boron in the surface. The stent can have an overall boron concentration of between 0.005 and 0.5 weight percent boron (e.g., between 0.01 and 0.1 weight percent boron). The boron enriched surface region can be at least 1 nanometer thick and/or no more than 5 microns thick (e.g, between 1 nanometer and 3 microns). In some embodiments, a surface region having a thickness between 1 nanometer and 3 microns can have a boron concentration of between 1 weight percent boron and 30 weight percent boron. The boron concentration can decrease in the thickness direction of the member from the boron enriched surface region to the bulk region. The depth of the boron penetration in the thickness direction of the member can be selected to ensure that the stent retains sufficient shape memory properties to enable it to perform the role of supporting a body passageway. In some embodiments, the addition of the boron will not alter the modulus of elasticity and/or the ultimate tensile strength of the member by more than five percent.

Implanted boron or boron ions can combine with the shape memory alloy of the stent to form boride intermetallic phases (e.g., TiB or NbB). The boride intermetallic phases can impart increased surface hardness and fatigue resistance to the stent. For example, implanting boron or boron ions into the surface of a Ni-Ti alloy stent can result in the formation of titanium boride intermetallic phases along the surface. By implanting boron or boron ions into the surface of other shape memory alloys, other boride intermetallic phases can be formed.

FIG. 2 illustrates a second exemplary stent 20 that includes a shape memory alloy having a boron enriched surface region 28. The boron enhanced surface region 28 can enhance the surface hardness and fatigue resistance of the stent 20.

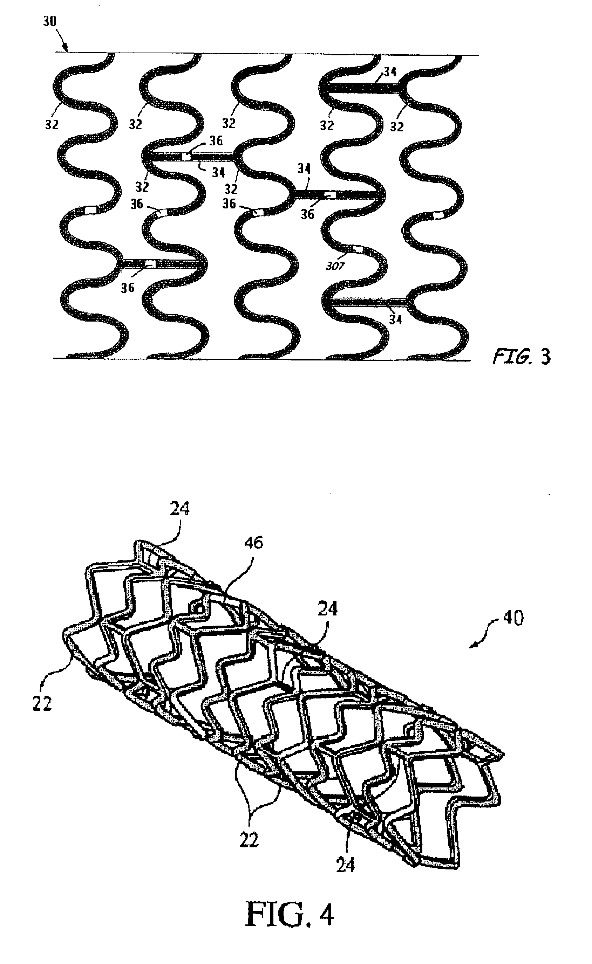

FIG. 3 illustrates an exemplary stent 30 including a plurality of bands 32 and a plurality of connectors 34. The bulk of stent 30 can include a shape memory alloy, such as a Ni-Ti alloy. The majority of the surface surfaces of these bands 32 and connectors 34 can be enriched with boron, which imparts enhanced surface hardness and fatigue resistance. Stent 30 can also include non-boron enriched portions 36. FIG. 4 also illustrates an exemplary stent 40 including bands 22, connectors 24 and non-boron enriched stent sections 46.

By selectively enriching the surface of stent 30 or stent 40 with boron, the cracking of the stent can occur in predetermined areas and in a predetermined pattern. For example, the non-boron enriched stent portions can be arranged to transition the stent from a closed stent structure to a set of separate rings or to a spiral shape. The cracking of predetermined stent portions can relieve strain in other areas of stent 30 or stent 40.

Boron can be implanted into the surface of the shape memory alloy of the stent by a variety of methods, including plasma ion immersion implantation (PIII) or beamline ion implantation of boron ions into the surface of the stent.

In some embodiments, the stent can be heat set into a desired expanded state and boron ions can be implanted into the surface of the stent. Shape setting can be achieved over a wide temperature range from 300°C to 900°C. For example, heat treating temperatures for a binary NiTi alloys are sometimes chosen in the narrower range of 325 to 525°C to achieve a combination of physical and mechanical properties (e.g., 510°C. For example, a binary NiTi alloy can be heat treated at 510°C for a time of between 5 minutes to 30 minutes.

In some embodiments the boron ion implantation can be preformed at a temperature up to about 250°C (e.g., between room temperature and 200°C). For example, the implantation process can be a room temperature process that does not alter the heat set of the stent. In some embodiments, a heated boron ion implantation process can be limited to no more than about 2 hours.

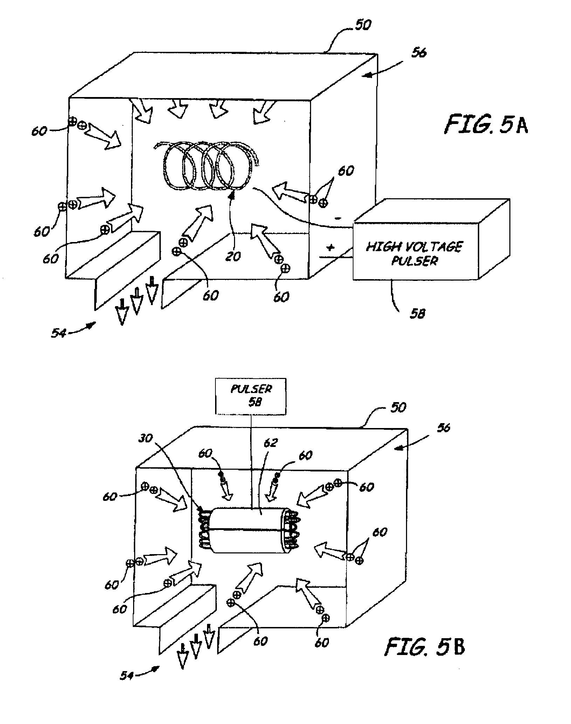

FIG. 5A illustrates an exemplary environment for performing PIII. In order to perform PIII, stent 20 is inserted into a chamber 50. Chamber 50 is a vacuum chamber created by vacuum 54 containing a plasma 56. Plasma 56 contains boron ions to be implanted into stent 20. Stent 20 is pulsed repeatedly with high negative voltages from pulser 58. As a result of the pulses of negative voltages, electrons are repelled away from stent 20 and positive boron ions 60 are attracted to the negatively charged stent 20. As a result, positive boron ions will strike all the surfaces of stent 20 and be embedded in and/or deposited onto stent 20. In some embodiments, one could change the electric field between the first pulse and the last pulse during the implantation process to control the implantation to crease a desired concentration gradient in the thickness direction of the stent. For example, one could gradually decrease the electric field during implantation to create a smooth transition between the boron enriched surface and the bulk region.

FIG. 5B also illustrates an exemplary environment for performing PIII on a selected portion of stent 30. Stent 30 is positioned within a shield 62, which blocks predetermined areas to result in selective non-boron enriched stent portions 36. Although shown as covering the majority of the stent 30, shield 62 in many embodiments could only cover smaller portions of the stent 30. The shield 62 would block portions of stent 30 so that ions from within chamber 50 would only be applied to the exposed portions of the stent 30. In some embodiments, shield 62 could be metal, wherein an electric contact would be formed between the shield 62 and stent 30 in order to provide the negative voltage pulses. In this embodiment, pulser 58 would be electrically coupled to the shield 62. Other suitable materials such as polymers could also be used as the shield 62.

Any of the stents illustrated in FIGS. 1-4 can further include a layer of carbon overlying the boron enriched surface region, carbides embedded within the boron enriched surface region, or a combination thereof. The carbon can be deposited and/or embedded by PIII to form an overlying layer of carbon, embedded carbides within the boron enriched surface, or a combination thereof. For example, a stent can include titanium carbides by using RF C₂H₂ PIII to deposit an amorphous hydrogenated carbon film onto a Ni-Ti alloy stent.

Stents 10, 20, 30, or 40 can be of any desired shape and size (e.g., superficial femoral artery stents, coronary stents, aortic stents, peripheral vascular stents, gastrointestinal stents, urology stents, and neurology stents). Depending on the application, the stent can have a diameter of between, for example, 1 mm to 46 mm. In certain embodiments, a coronary stent can have an expanded diameter of from 2 mm to 6 mm. In some embodiments, a peripheral stent can have an expanded diameter of from 5 mm to 24 mm. In certain embodiments, a gastrointestinal and/or urology stent can have an expanded diameter of from 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm.

In some embodiments, the stent 10, 20, 30, or 40 can be a superficial femoral artery stent. Superficial femoral artery stents can be subject to repeated large strains during use, for example due to the bending of a knee. The strain resistance of a superficial femoral artery stent can be improved by implanting boron into the surface of a shape memory alloy making up the superficial femoral artery stent.

In use, a stent can be used, e.g., delivered and expanded, using a catheter delivery system. Catheter systems are described in, for example, Wang U.S. 5,195,969, Hamlin U.S. 5,270,086, and Raeder-Devens, U.S. 6,726,712. Stents and stent delivery are also exemplified by the Sentinol ^{®} system, available from Boston Scientific Scimed, Maple Grove, MN.

In some embodiments, stents can also be a part of a covered stent or a stent-graft. In other embodiments, a stent can include and/or be attached to a biocompatible, nonporous or semi-porous polymer matrix made of polytetrafluoroethylene (PTFE), expanded PTFE, polyethylene, urethane, or polypropylene.

In some embodiments, stents can also include a releasable therapeutic agent, drug, or a pharmaceutically active compound, such as described in U.S. Patent No. 5,674,242, U.S. Patent No. 2003/003220, and U.S. Patent No. 2003/185895. The therapeutic agents, drugs, or pharmaceutically active compounds can include, for example, anti-thrombogenic agents, antioxidants, anti-inflammatory agents, anesthetic agents, anti-coagulants, and antibiotics.

In some embodiments, stents can be formed by fabricating a wire including a boride enhanced surface, and knitting and/or weaving the wire into a tubular member.

## Claims

1. An endoprosthesis comprising
a member capable of supporting a body passageway, the member having a surface region overlying a bulk region, and comprising a shape memory alloy and boron, wherein the concentration of boron in the surface region is greater than the concentration of boron in the bulk region and wherein the member capable of supporting a body passageway is a stent.

2. The endoprosthesis of claim 1, wherein the shape memory alloy comprises titanium, niobium, or a combination thereof.

3. The endoprosthesis of claim 2, wherein the shape memory alloy comprises a Ni-Ti alloy.

4. The endoprosthesis of claim 1, wherein the surface region comprises boride intermetallic phases.

5. The endoprosthesis of claim 4, wherein the surface region comprises titanium boride phases, niobium boride phases, or a combination thereof.

6. The endoprosthesis of one of the preceeding claims, wherein the concentration of boron in the member decreases in the thickness direction from the surface region to the bulk region.

7. The endoprosthesis of one of the preceeding claims, further comprising a carbon layer overlying the surface region, carbides embedded within the surface region, or a combination thereof.

8. The endoprosthesis of claim 7, comprising titanium carbide embedded in the surface region.

9. The endoprosthesis of claim 1, wherein the stent is a self-expanding stent.

10. The endoprosthesis of claim 1, wherein the stent is a superficial femoral artery stent.

11. The endoprosthesis of claim 1, wherein the member further comprises portions comprising a shape memory alloy but lacking boron.

12. A method of producing an endoprosthesis,
the endoprosthesis comprising
a member capable of supporting a body passageway, the member having a surface region overlying a bulk region, and comprising a shape memory alloy and boron,
wherein the concentration of boron in the surface region is greater than the concentration of boron in the bulk region, and
wherein the member capable of supporting a body passageway is a stent;
the method comprising the steps of
heat setting an endoprosthesis comprising a shape memory alloy into a predetermined expanded state; and
implanting boron ions into the surface of the endoprosthesis.

13. The method of claim 12, wherein the boron ions are selectively implanted into the surface of the heat set endoprosthesis to produce portions that include a surface comprising the shape memory alloy and boron, and portions that include a surface comprising the shape memory alloy but lacking boron.

14. The method of claim 12 or 13, wherein the boron ions are implanted by plasma ion immersion implantation.

15. The method of one of claims 12 to 14, further comprising incorporating carbon into or onto the surface of the heat set endoprosthesis.

## Patentansprüche

1. Eine Endoprothese umfassend:
Ein Bauteil, geeignet einen Körperdurchgang zu stützen, wobei das Bauteil einen Oberflächenbereich hat, der einen Massebereich überdeckt, und
wobei das Bauteil eine Formgedächtnis-Legierung und Bor umfasst, wobei die Konzentration an Bor im Oberflächenbereich größer ist als die Konzentration an Bor im Massebreich, und wobei das Bauteil, das geeignet ist einen Körperdurchgang zu stützen, ein Stent ist.

2. Die Endoprothese gemäß Anspruch 1, wobei die Formgedächtnis-Legierung Titan, Niob oder eine Kombination davon umfasst.

3. Die Endoprothese gemäß Anspruch 2, wobei die Formgedächtnis-Legierung eine Ni-Ti Legierung umfasst.

4. Die Endoprothese gemäß Anspruch 1, wobei der Oberflächenbereich Borid-haltige intermetallische Phasen umfasst.

5. Die Endoprothese gemäß Anspruch 4, wobei der Oberflächenbereich Titanborid-Phasen, Niobborid-Phasen, oder eine Kombination davon umfasst.

6. Die Endoprothese gemäß einem der vorangehenden Ansprüche, wobei die Konzentration an Bor im Bauteil in der Dickerichtung vom Oberflächenbereich zum Massebereich abfällt.

7. Die Endoprothese gemäß einem der vorangehenden Ansprüche weiter umfassend: Eine Carbonschicht, den Oberflächenbereich überdeckend, Carbide, eingebettet in den Oberflächenbereich, oder eine Kombination davon.

8. Die Endoprothese gemäß Anspruch 7, ein Titan-Carbid umfassend, das in den Oberflächenbereich eingebettet ist.

9. Die Endoprothese gemäß Anspruch 1, wobei der Stent ein selbstexpandierender Stent ist.

10. Die Endoprothese gemäß Anspruch 1, wobei der Stent ein Stent für die superficiale femorale Arterie ist.

11. Die Endoprothese gemäß Anspruch 1, wobei das Bauteil weiter umfasst:
Teile, die eine Formgedächtnis-Legierung umfassen, aber kein Bor enthalten.

12. Ein Verfahren zur Herstellung einer Endoprothese, die Endoprothese umfassend:
Ein Bauteil, geeignet einen Körperdurchgang zu stützen, wobei das Bauteil einen Oberflächenbereich hat, der einen Massebereich überdeckt, und wobei das Bauteil eine Formgedächtnis-Legierung und Bor umfasst, wobei die Konzentration an Bor im Oberflächenbereich größer ist als die Konzentration an Bor im Massebereich, und wobei das Bauteil, das geeignet ist einen Körperdurchgang zu stützen, ein Stent ist;
Das Verfahren folgende Schritte umfassend:
Wärmefixieren einer Endoprothese, die eine Formgedächtnis-Legierung in einem vorbestimmten expandierten Zustand umfasst; und
Implantieren von Borionen in die Oberfläche der Endoprothese.

13. Das Verfahren gemäß Anspruch 12, wobei die Borionen selektiv in die Oberfläche der wärmefixierten Endoprothese implantiert sind, um Teile zu produzieren, die eine Oberfläche beinhalten, die die Formgedächtnis-Legierung und Bor umfasst, und um Teile zu produzieren, die eine Oberfläche beinhalten, die die Formgedächtnis-Legierung umfasst, aber kein Bor enthält.

14. Das Verfahren gemäß Anspruch 12 oder 13, wobei die Borionen durch Plasmaionen-Immersionsimplantation implantiert werden.

15. Das Verfahren gemäß einem der Ansprüche 12 bis 14, weiter umfassend:
Einbringung von Carbon in oder auf die Oberfläche der wärmefixierten Endoprothese.

## Revendications

1. Endoprothèse comprenant
un organe capable de supporter un passage corporel l'organe ayant une région de surface recouvrant une région massive, et comprenant un alliage à mémoire de forme et du bore, la concentration de bore dans la région de surface étant supérieure à la concentration de bore dans la région massive et l'organe capable de supporter un passage corporel étant un stent.

2. Endoprothèse selon la revendication 1, dans laquelle l'alliage à mémoire de forme comprend du titane, du niobium ou une combinaison de ceux-ci.

3. Endoprothèse selon la revendication 2, dans laquelle l'alliage à mémoire de forme comprend un alliage Ni-Ti.

4. Endoprothèse selon la revendication 1, dans laquelle la région de surface comprend des phases intermétalliques à base de borure.

5. Endoprothèse selon la revendication 4, dans laquelle la région de surface comprend des phases de borure de titane, des phases de borure de niobium, ou une combinaison de celles-ci.

6. Endoprothèse selon l'une quelconque des revendications précédentes, dans laquelle la concentration de bore dans l'organe diminue dans la direction de l'épaisseur depuis la région de surface jusqu'à la région massive.

7. Endoprothèse selon l'une quelconque des revendications précédentes, comprenant en outre une couche de carbone recouvrant la région de surface, des carbures noyés à l'intérieur de la région de surface, ou une combinaison de ceux-ci.

8. Endoprothèse selon la revendication 7, comprenant du carbure de titane noyé dans la région de surface.

9. Endoprothèse selon la revendication 1, dans laquelle le stent est un stent auto-expanseur.

10. Endoprothèse selon la revendication 1, dans laquelle le stent est un stent de l'artère fémorale superficielle.

11. Endoprothèse selon la revendication 1, dans laquelle l'organe comprend en outre des portions comprenant un alliage à mémoire de forme mais dépourvues de bore.

12. Procédé de production d'une endoprothèse,
l'endoprothèse comprenant
un organe capable de supporter un passage corporel l'organe ayant une région de surface recouvrant une région massive, et comprenant un alliage à mémoire de forme et du bore,
la concentration de bore dans la région de surface étant supérieure à la concentration de bore dans la région massive et
l'organe capable de supporter un passage corporel étant un stent ;
le procédé comprenant les étapes consistant à:
thermofixer une endoprothèse comprenant un alliage à mémoire de forme dans un état expansé prédéterminé ; et
implanter des ions bore dans la surface de l'endoprothèse.

13. Procédé selon la revendication 12, dans lequel les ions bore sont implantés de manière sélective dans la surface de l'endoprothèse thermofixée pour produire des portions qui comprennent une surface comprenant l'alliage à mémoire de forme et du bore, et des portions qui comprennent une surface comprenant l'alliage à mémoire de forme mais dépourvues de bore.

14. Procédé selon la revendication 12 ou 13, dans lequel les ions bore sont implantés par implantation d'ions par immersion dans un plasma.

15. Procédé selon l'une quelconque des revendications 12 à 14, comprenant en outre l'incorporation de carbone dans ou sur la surface de l'endoprothèse thermofixée.
